# EUROPEAN PATENT APPLICATION

(11) **EP 3 187 109 A1**
(43) Date of publication of application: **05.07.2017**
(21) Application number: 15835535.4
(22) Date of filing: 19.08.2015
(51) Int. Cl.: A61B 5/0404, A61B 5/0205, A61B 5/0245, A61B 5/0402

(54) **BIOLOGICAL INFORMATION DETECTION DEVICE**

(30) Priority: 27.08.2014 JP 2014172689
(71) Applicant: Seiko Epson Corporation, Tokyo 160-8801 (JP)
(72) Inventor: WATANABE, Shinichiro, Suwa-shi Nagano 392-8502 (JP)
(74) Representative: Hoffmann Eitle
(86) International application number: PCT/JP2015/004111
(87) International publication number: WO 2016/031179

(57) **Abstract**

A biological information detection device which detects an arrhythmia on the basis of pulse wave information, performs control on measurement of an electrocardiogram on the basis of the detection result, and thereby restrains failure to record the electrocardiogram at the time of occurrence of an arrhythmia, or the like, is to be provided.

A biological information detection device 100 includes a pulse wave information measuring unit 110 which measures pulse wave information, an electrocardiogram measuring unit 120 which measures an electrocardiogram, and a control unit 130 which performs at least one of power supply control on the electrocardiogram measuring unit 120, storage control for electrocardiogram measurement result information measured by the electrocardiogram measuring unit 120, and notification control to output notification information about the measurement of the electrocardiogram using the electrocardiogram measuring unit 120, to a user, if it is determined that an arrhythmia is detected on the basis of detection result information obtained by analysis processing based on the pulse wave information.

## Description

### Technical Field

The present invention relates to a biological information detection device and the like.

### Background Art

In monitoring the state of a heart disease patient or the like, the patient or subject is made to wear a Holter electrocardiograph for 24 hours or 48 hours, and electrocardiogram data in everyday life is thus observed so as to give a definitive diagnosis. However, with respect to attacks of arrhythmia, attacks at a low frequency such as a few times a month or week or so are conceivable. In the case of such attacks, the period of occurrence of arrhythmia and the period when the Holter electrocardiograph is worn may not overlap with each other and symptoms of the heart disease such as arrhythmia may not be able to be recorded.

Also, with a Holter electrocardiograph, a plurality of electrodes needs to be attached to the chest of a human body, and the problem of skin rashes arises due to the electrodes and tapes for holding the electrodes. Therefore, it is difficult to wear the Holter electrocardiograph for a long period (a period long enough to prevent the possibility of overlooking attacks at a low frequency, for example, approximately three to ten days). In recent years, though there is an electrocardiograph capable of recording for 40 days, the problem of skin rashes has not been solved and it is not realistic to use this electrocardiograph continuously for a long time.

Meanwhile, PTL 1 discloses a device which enables the patient him/herself to record an electrocardiogram by holding a portable electrocardiograph on his/her chest and pressing a recording button (event button) when the patient perceives a symptom. With respect to the device of PTL 1, particularly in the electrocardiography carried out using a main body electrode part, there is no need to hold the electrode with a tape or the like and therefore the problem of skin rashes does not arise.

Also, PTL 2 discloses a wristwatch-type electrocardiograph. With the device of PTL 2, the device is fixed to the user with a support member such as a band similarly to a wristwatch, and therefore the problem of skin rashes or the like tends not to occur.

### Citation List

### Patent Literature

PTL 1: JP-A-2009-82364
PTL 2: JP-A-60-103935

### Summary of Invention

### Technical Problem

As described above, electrocardiographs such as the Holter electrocardiograph are not suitable for long-term monitoring because of skin rashes caused by electrodes and troublesomeness of electrode cords or the like. With the devices disclosed in PTL 1 and PTL 2, since no electrodes are attached, the problem of skin rashes or the like tends not to occur. However, conversely, in the techniques of PTL 1 and PTL 2, the measurement itself cannot be carried out unless the user (patient, wearer) intentionally attempts to measure an electrocardiogram. That is, these are devices on which the patient him/herself carries out an electrocardiogram recording operation when the patient perceives an arrhythmia attack.

Meanwhile, there are many asymptomatic patients who themselves do not perceive an arrhythmia attack. In such cases, the devices of PTL 1 and the like may not be useful for the detection of a heart disease such as arrhythmia because the measurement of an electrocardiogram is not carried out in the first place.

According to some embodiments of the invention, a biological information detection device can be provided which detects an arrhythmia on the basis of pulse wave information and carries out control on measurement of an electrocardiogram on the basis of the result of the detection, thereby preventing failure to record an electrocardiogram at the time of occurrence of an arrhythmia.

### Solution to Problem

An embodiment of the invention relates to a biological information detection device including: a pulse wave information measuring unit which measures pulse wave information; an electrocardiogram measuring unit which measures an electrocardiogram; and a control unit which performs at least one of power supply control on the electrocardiogram measuring unit, storage control for electrocardiogram measurement result information measured by the electrocardiogram measuring unit, and notification control to output notification information about the measurement of the electrocardiogram using the electrocardiogram measuring unit, to a user, if it is determined that an arrhythmia is detected on the basis of detection result information obtained by analysis processing based on the pulse wave information.

According to the embodiment of the invention, control on the measurement of the electrocardiogram is carried out on the basis of the result of analysis processing using pulse wave information. Thus, even in the case of an arrhythmia or the like without any subjective symptoms, it is possible to properly measure the electrocardiogram, or the like.

Also, in the embodiment of the invention, the device may include an output unit which outputs the electrocardiogram measurement result information, and the output unit may output the electrocardiogram measurement result information in association with the detection result information of the arrhythmia based on the pulse wave information.

Thus, it is possible to output additional information in association with the electrocardiogram measurement result information, or the like.

Also, in the embodiment of the invention, the output unit may output body movement information of the user acquired by a body movement sensor or action information of the user determined on the basis of the body movement information, in association with the electrocardiogram measurement result information.

Thus, it is possible to output additional information in association with the electrocardiogram measurement result information, or the like.

Also, in the embodiment of the invention, the output unit may output autonomic nerve activity information acquired on the basis of the pulse wave information, in association with the electrocardiogram measurement result information.

Thus, it is possible to output additional information in association with the electrocardiogram measurement result information, or the like.

Also, in the embodiment of the invention, the control unit may perform at least one of the power supply control, the storage control, and the notification control, using a detection result of atrial fibrillation as the detection result information of the arrhythmia.

Thus, it is possible to detect atrial fibrillation as the arrhythmia and perform control relating to the measurement of the electrocardiogram based on the detection result information.

Also, in the embodiment of the invention, the control unit may perform at least one of the power supply control, the storage control, and the notification control, using a detection result of tachycardia as the detection result information of the arrhythmia.

Thus, it is possible to detect tachycardia as the arrhythmia and perform control relating to the measurement of the electrocardiogram based on the detection result information.

Also, in the embodiment of the invention, the control unit may perform at least one of the power supply control, the storage control, and the notification control, using a detection result of bradycardia as the detection result information of the arrhythmia.

Thus, it is possible to detect bradycardia as the arrhythmia and perform control relating to the measurement of the electrocardiogram based on the detection result information.

Also, in the embodiment of the invention, the control unit may perform the power supply control to start supplying electric power to the electrocardiogram measuring unit if it is determined that the arrhythmia is detected on the basis of the pulse wave information.

Thus, it is possible to start supplying electric power or the like, triggered by the determination that the arrhythmia is detected.

Also, in the embodiment of the invention, the control unit may perform the storage control to start storing the electrocardiogram measurement result information in a storage unit if it is determined that the arrhythmia is detected on the basis of the pulse wave information.

Thus, it is possible to start storing the electrocardiogram measurement result information or the like, triggered by the determination that the arrhythmia is detected.

Also, in the embodiment of the invention, the control unit may perform the notification control to start outputting the notification information for prompting the measurement of the electrocardiogram using the electrocardiogram measuring unit, if it is determined that the arrhythmia is detected on the basis of the pulse wave information.

Thus, it is possible to start the notification control for prompting the measurement of the electrocardiogram or the like, triggered by the determination that the arrhythmia is detected.

Also, in the embodiment of the invention, the control unit may halt control on the supply of the electric power, halt control on the storage of the electrocardiogram measurement result information, or halt control on the output of the notification information, on the basis of the detection result information of the arrhythmia.

Thus, it is possible to perform halt control on various operations on the basis of the detection result information of the arrhythmia.

Also, in the embodiment of the invention, the device may include an analysis processing unit which performs the analysis processing based on the pulse wave information and finds the detection result information of the arrhythmia.

Thus, it is possible to perform the arrhythmia analysis processing based on the pulse wave information within the biological information detection device.

Also, in the embodiment of the invention, the device may include a receiving unit which receives the detection result information of the arrhythmia based on the pulse wave information, and the control unit may perform at least one of the power supply control, the storage control, and the notification control, on the basis of the detection result information that is received.

Thus, it is possible to acquire the detection result information indicating the result of analysis processing carried out by another processing device or the like, and perform control relating to the measurement of the electrocardiogram or the like.

Also, in the embodiment of the invention, the device may include a case unit which accommodates the pulse wave information measuring unit, the electrocardiogram measuring unit, and the control unit. A first electrode for electrocardiogram measurement may be provided on a surface on the side opposite to a subject, of the case unit. A second electrode for electrocardiogram measurement may be provided on a surface on the side of the subject, of the case unit.

Thus, it is possible to properly arrange the electrodes for electrocardiogram measurement in the case unit of the biological information detection device, or the like.

Also, in the embodiment of the invention, the case unit may have a top case and a bottom case. The first electrode may be provided on a surface on the side opposite to the subject, of the top case. The second electrode may be provided on a surface on the side of the subject, of the bottom case.

Thus, it is possible to form the biological information detection device with the top case and the bottom case, and also arrange one of the electrodes for electrocardiogram measurement on the top case and arrange the other one on the bottom case, or the like.

Also, in the embodiment of the invention, the device may include a light emitting unit which casts light on a subject and a light receiving unit which receives light from the subject, and a detection window for casting the light from the light emitting unit onto the subject and receiving the light from the subject. The pulse wave information measuring unit may measure the pulse wave information on the basis of a signal from the light receiving unit. An electrode for measuring the electrocardiogram may be provided in a peripheral part of the detection window.

Thus, in the case of using a sensor having a light emitting unit and a light receiving unit, it is possible to cast and receive proper light by providing the detection window, and it is possible to properly set the relation between the detection window and the electrode, or the like.

Also, in the embodiment of the invention, the detection window may have a protruding part for applying a pushing pressure to the subject. The device may include a pushing pressure restraining part which restrains the pushing pressure applied to the subject by the protruding part. The electrode may be provided on the pushing pressure restraining part.

Thus, by providing the protruding part and the pushing pressure restraining part, it is possible to apply a proper and stable pushing pressure to the subject. Also, by providing the electrode on the pushing pressure restraining part, it is possible to bring the electrode and the subject in stable contact, or the like.

### Brief Description of Drawings

[FIG. 1] FIG. 1 shows an example of the configuration of a biological information detection device according to an embodiment.
[FIG. 2] FIG. 2 shows an example of the detailed configuration of the biological information detection device according to the embodiment.
[FIG. 3] FIGS. 3(A) to 3(C) show a specific example of the implementation of a system including the biological information detection device.
[FIG. 4] FIG. 4 (A) is a view illustrating pulse AC signal and pulse interval. FIG. 4(B) is a view illustrating the processing of finding LF and HF from pulse wave information.
[FIG. 5] FIG. 5 shows an example of the shape of the biological information detection device.
[FIG. 6] FIGS. 6 (A) and 6 (B) show an example of the shape of the biological information detection device.
[FIG. 7] FIGS. 7 (A) and 7 (B) show an example of the posture of the user at the time of measuring an electrocardiogram.
[FIG. 8] FIG. 8 is a cross-sectional view showing the structure of a detection window and its peripheries.
[FIG. 9] FIG. 9 is a view for explaining a change in signal sensitivity and noise sensitivity due to a pushing pressure.
[FIG. 10] FIG. 10 is an explanatory view of a change in pushing pressure with respect to application of load in the case where a pushing pressure restraining part is provided.
[FIG. 11] FIG. 11 shows an example of the detailed configuration of the biological information detection device according to the embodiment.
[FIG. 12] FIG. 12 shows an example of the detailed configuration of an analysis processing unit
[FIG. 13] FIGS. 13(A) and 13(B) are graphs showing the result of frequency analysis processing based on the electrocardiogram RR interval.
[FIG. 14] FIGS. 14(A) and 14(B) are graphs showing the result of frequency analysis processing based on the average pulse wave RR interval.
[FIG. 15] FIG. 15 is a view for explaining a period determined as atrial fibrillation.
[FIG. 16] FIG. 16 is a flowchart for explaining atrial fibrillation determination processing (analysis processing).

### Description of Embodiments

Hereinafter, an embodiment will be described. However, the embodiment described below should not unduly limit the contents of the invention described in the claims. Also, not all the configurations described in the embodiment are essential components of the invention.

### 1. Technique in This Embodiment

First, the technique in this embodiment will be described. As described above, arrhythmia is known as one of heart-related diseases. Arrhythmias include tachyarrhythmia, in which the pulse rate tends to increase, and bradyarrhythmia, in which conversely the pulse rate tends to drop. Tachyarrhythmia can be further classified into supraventricular tachycardia, atrial flutter, atrial fibrillation and the like.

It is known that any type of arrhythmia can be detected and classified by measuring an electrocardiogram. Specifically, various kinds of information found from the electrocardiogram such as the regularity of the RR interval, the presence/absence of P-wave and F-wave, and the PQ interval are to be used. However, this is a known technique and therefore will not be described further in detail.

With respect to attacks of arrhythmia, attacks particularly at a low frequency such as a few times a month or week or so are conceivable. In the case of such attacks, the period of occurrence of arrhythmia and the period when the Holter electrocardiograph is worn may not overlap with each other and symptoms of the heart disease such as arrhythmia may not be able to be recorded. On the other hand, if the Holter electrocardiograph is worn over a span of three to ten days, the period of occurrence of arrhythmia and the period when the Holter electrocardiograph is worn overlap with each other, that is, it is possible to properly measure an electrocardiogram and properly detect the arrhythmia.

However, with a common electrocardiograph such as the Holter electrocardiograph, electrodes need to be fixed with tapes or the like. Therefore, the problem of skin rashes or the like arises, thus making it difficult to wear the device for a long time and leaving the risk of failure to detect the arrhythmia.

Meanwhile, with the devices (electrocardiographs) disclosed in PTL 1 and PTL 2, when there is a subjective symptom of arrhythmia, the user brings the electrode in contact with a predetermined part of him/herself and thus measures an electrocardiogram. In this way, the user does not have to constantly wear the electrode, the problem of skin rashes or the like does not arise. Moreover, since measurement is carried out when there is a subjective symptom, it seems possible to acquire a desired electrocardiogram, that is, an electrocardiogram that is useful to determine a heart disease such as arrhythmia (in a narrow sense, a diagnosis by a doctor) .

However, the case where "since measurement is carried out when there is a subjective symptom, a desired electrocardiogram can be acquired" is based on the assumption that the user is able to be aware of the occurrence of an arrhythmia. Since there are arrhythmias without any subjective symptoms (for example, asymptomatic atrial fibrillation), such an assumption may not hold in some cases. If there are no subjective symptoms, the user will not carry out measurement using the electrocardiograph in the first place. Therefore, in practice, despite the occurrence of an arrhythmia, the electrocardiogram during that period will not be measured.

Of arrhythmias without subjective symptoms, particularly atrial fibrillation tends to cause a thrombus and there is a risk of the thrombus shifting to the brain and blocking blood vessels in the brain, thus causing serious cardiogenic cerebral infarction. Therefore, in view of proper detection of an arrhythmia and prevention of severe symptoms, the situation where the measurement of the electrocardiogram is not carried out because of the absence of subjective symptoms must be restrained as much as possible.

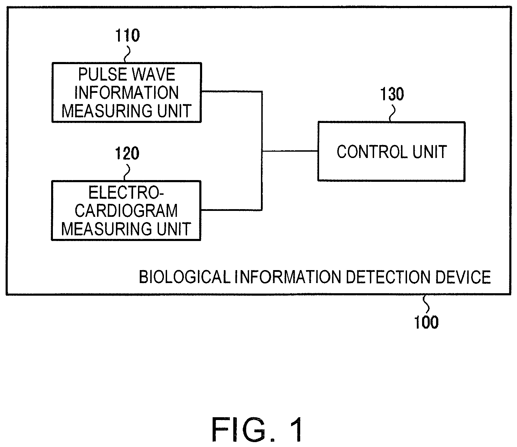

Thus, the present applicant proposes a technique for determining an arrhythmia on the basis of pulse wave information. Specifically, a biological information detection device 100 according to this embodiment includes a pulse wave information measuring unit 110 which measures pulse wave information, an electrocardiogram measuring unit 120 which measures an electrocardiogram, and a control unit 130 which performs at least one of power supply control on the electrocardiogram measuring unit 120, storage control on electrocardiogram measurement result information measured by the electrocardiogram measuring unit 120, and notification information relating to the measurement of the electrocardiogram using the electrocardiogram measuring unit 120, if it is determined that an arrhythmia is detected on the basis of detection result information obtained by analysis processing based on the pulse wave information, as shown in FIG. 1.

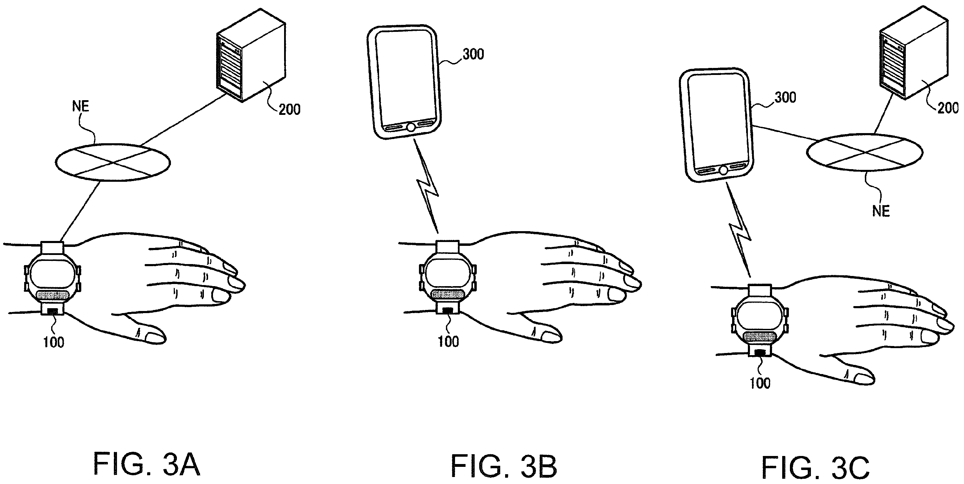

As will be described in detail later, a pulse wave sensor used to measure pulse wave information can be implemented, for example, by a photoelectric sensor. The specific form of the biological information detection device 100 varies, depending on whether a light receiving unit receives transmitted light or reflected light. However, in any case, the biological information detection device 100 may be fixed to a predetermined part of the user with a support member such as a band. The predetermined part in this case may be a wrist as described later with reference to FIG. 3(A) or the like, or may be other parts such as a finger, neck, or ankle.

Therefore, compared with an electrocardiogram measuring device such as the Holter electrocardiogram, the biological information detection device 100 according to the embodiment tends not to cause the problem of skin rashes or like and can be worn continuously for a long period. Therefore, it is easy to acquire pulse wave information continuously over a period of three to ten days or so. Even in the state where the frequency of occurrence of arrhythmia is low, it is possible to restrain the possibility of failure to detect the arrhythmia in arrhythmia detection processing based on the pulse wave information.

Also, since an increase (tachycardia) or decrease (bradycardia) in pulse rate, an irregularity in heartbeat interval (pulse interval) or the like occurs as symptom of arrhythmia, it is possible to detect many types of arrhythmias by using pulse wave information even if the user him/herself has no subjective symptoms. To summarize the above, it can be said that the biological information detection device 100 according to the embodiment has no problem with low frequencies of occurrence of arrhythmia or with the absence of subjective symptoms during the occurrence and can properly detect the occurrence, using pulse wave information.

However, while it is known from an experiment made by the applicant that an arrhythmia can be detected with a certain accuracy using pulse wave information, an electrocardiogram is used in the current medical definitive diagnosis of arrhythmia and the accuracy of determination can be increased as well. Therefore, it is also important to measure an electrocardiogram at the time of the occurrence of arrhythmia.

Thus, in the embodiment, the result of detection of an arrhythmia based on pulse wave information is used as a trigger for the control relating to electrocardiogram measurement. As will be described in detail later, at least one of the power supply control on the electrocardiogram measuring unit 120, the storage control on the measured electrocardiogram, and the notification control to the user may be performed. In this way, it is possible to measure and store an electrocardiogram at a proper timing, using the result of detection of an arrhythmia based on pulse wave information. Therefore, it is possible to restrain failure to measure the electrocardiogram or the like, irrespective of whether the arrhythmia accompanies a subjective symptom or not.

Hereinafter, a specific example of the configuration of the biological information detection device according to the embodiment will be described and subsequently a control method using the result of detection of an arrhythmia based on pulse wave information will be described. Finally, a specific example of a method for detecting an arrhythmia on the basis of pulse wave information will be described with several patterns.

### 2. Example of Configuration of Biological Information Detection Device

Next, an example of the configuration of the biological information detection device 100 will be described. First, an example of the system configuration (functional block diagram) will be described, and subsequently a specific example of the shape will be described. Also, an example of the system in the case where the biological information detection device 100 is linked with other device will be described as well.

### 2.1 Functional Block Diagram

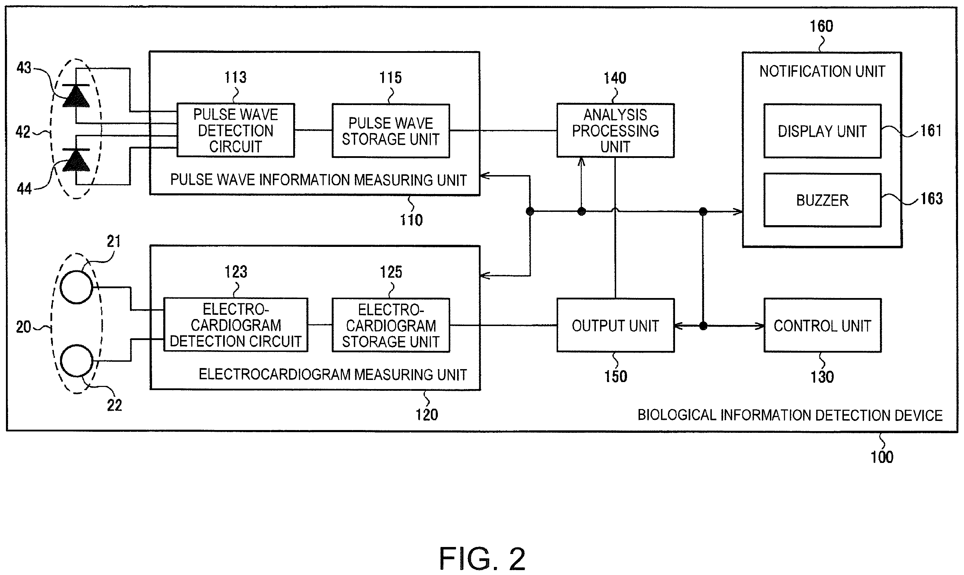

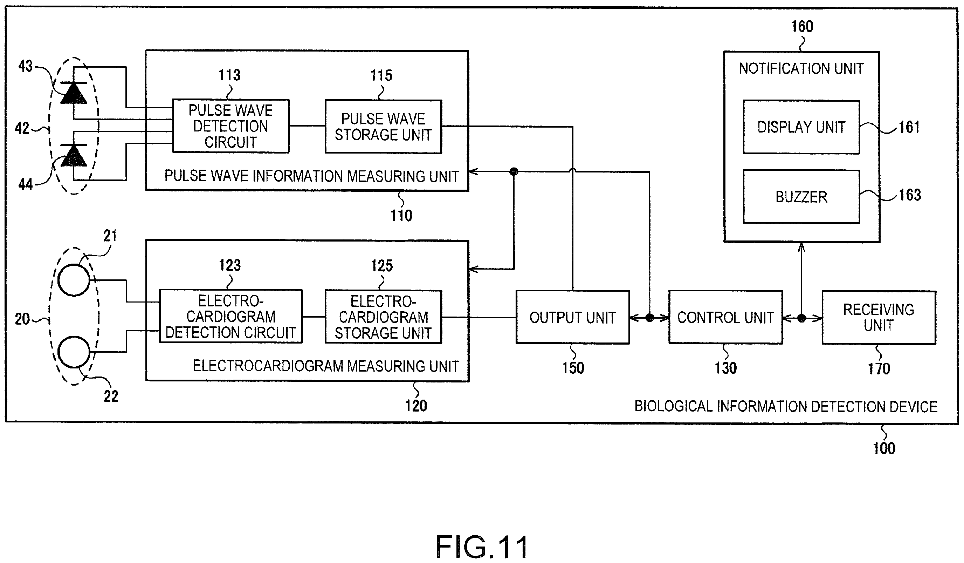

FIG. 2 shows an example of the detailed system configuration of biological information detection device 100 according to the embodiment. As shown in FIG. 2, the biological information detection device 100 includes a pulse wave sensor 42, an electrode section 20, the pulse wave information measuring unit 110, the electrocardiogram measuring unit 120, the control unit 130, an analysis processing unit 140, an output unit 150, and a notification unit 160. However, the configuration of the biological information detection device 100 is not limited to FIG. 2 and can be implemented with various modifications such as omitting a part of these components or adding another component or the like. Also, the possibility of implementation with modifications is similarly applied to the configuration of each part included in FIG. 2 and the illustrations showing other examples of the system configuration such as FIG. 11.

The pulse wave sensor 42 is a sensor for detecting pulse wave signals. For example, a photoelectric sensor including a light emitting unit 43 and a light receiving unit 44 is conceivable. It is known that the pulse wave sensor 42 can be implemented by various sensors such as a photoelectric sensor or other forms of sensors (for example, an ultrasonic sensor). These sensors can be broadly applied to the pulse wave sensor 42 in the embodiment.

The pulse wave information measuring unit 110 includes a pulse wave detection circuit 113 and a pulse wave information storage unit 115. The pulse wave detection circuit 113 is connected to the pulse wave sensor 42 and detects (acquires) pulse wave information on the basis of sensor information from the pulse wave sensor 42. The pulse wave information storage unit 115 stores the pulse wave information detected by the pulse wave detection circuit 113. The functions of the pulse wave information storage unit 115 are implemented by a memory such as a RAM or by an HDD (hard disk drive) or the like.

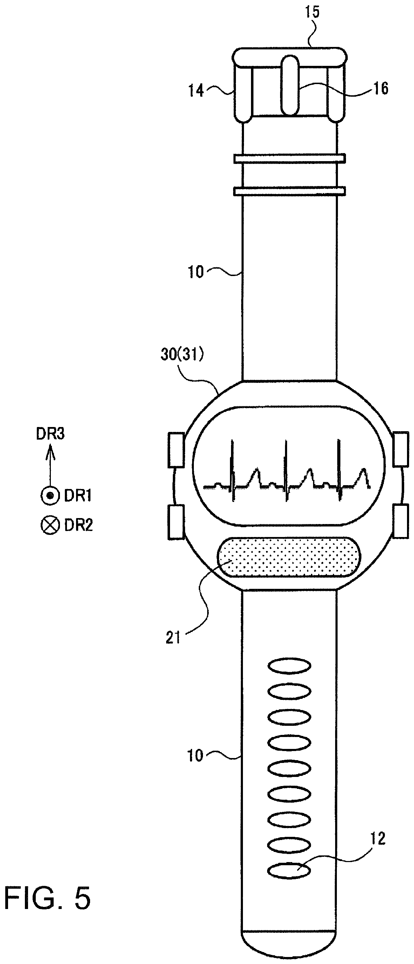

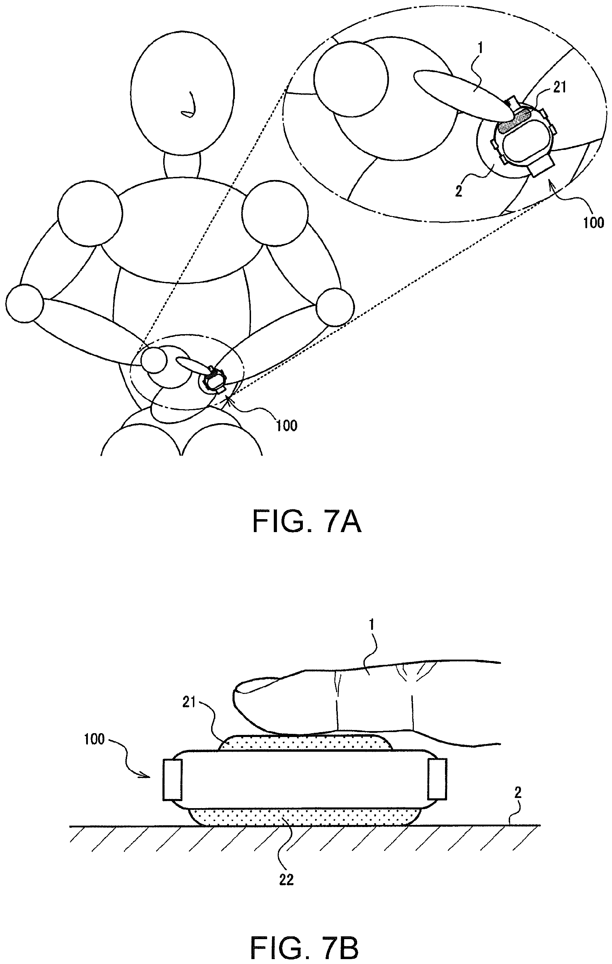

The electrode section 20, in a narrow sense, includes a first electrode 21 and a second electrode 22. The respective electrode parts are insulated from each other inside the biological information detection device 100. At the time of measuring an electrocardiogram, the first electrode 21 comes in contact with a part of the user on the side of a first direction with reference to the heart of the user, and the second electrode 22 comes in contact with a part of the user on the side of a second direction opposite to the side of the first direction. In the case where the biological information detection device 100 has the shape shown in FIG. 5 as described later and the biological information detection device 100 is mounted on the left arm of the user, the user is in the state shown in FIGS. 7 (A) and 7(B) at the time of electrocardiogram measurement and therefore the first electrode 21 comes in contact with a right finger 1 or the like of the user and the second electrode 22 comes in contact with a left wrist 2 of the user. That is, in the example of FIGS. 7(A) and 7(B), the first direction is the direction to the right of the heart and the second direction is the direction to the left of the heart when the front direction of the user is taken as a reference.

The electrocardiogram measuring unit 120 includes an electrocardiogram detection circuit 123 and an electrocardiogram storage unit 125. The electrocardiogram detection circuit 123 is connected to the electrode section 20 and measures (detects) an electrocardiogram on the basis of an electrical signal from the electrode section 20. The electrocardiogram storage unit 125 stores the electrocardiogram (electrocardiogram measurement result information) detected by the electrocardiogram detection circuit 123. The functions of the electrocardiogram storage unit 125 can be implemented by a memory such as a RAM or by an HDD (hard disk drive) or the like. Also, while the pulse wave information storage unit 115 and the electrocardiogram storage unit 125 are shown as separate blocks in FIG. 2, these may be implemented by a single memory or the like.

The control unit 130 performs various kinds of control on the basis of the result of analysis (result of detection) of an arrhythmia based on pulse wave information. Specifically, the control unit 130 may perform at least one of power supply control, storage control, and notification control. The power supply control is the control on power supply to the electrocardiogram measuring unit 120 from a battery, not illustrated. The storage control is the control to decide whether the electrocardiogram outputted from the electrocardiogram detection circuit 123 is to be stored in the electrocardiogram storage unit 125 or not. The notification control is the control to decide the start and end timings of the notification to the user from the notification unit 160, or specific contents of the notification. Details of the control contents by the control unit 130 will be described later.

Meanwhile, the analysis processing unit 140 reads out the pulse wave information stored in the pulse wave information storage unit 115 and performs arrhythmia analysis processing. The functions of this analysis processing unit 140 can be implemented by various processors (CPU or the like), hardware such as ASIC (gate array or the like), a program and the like. Details of the arrhythmia analysis processing will be described later.

In the example of FIG. 2, the biological information detection device 100 includes the analysis processing unit 140 which performs analysis processing based on pulse wave information and thus finds detection result information of arrhythmia. In this case, the arrhythmia analysis processing can be executed inside the biological information detection device 100.

The output unit 150 reads out the electrocardiogram stored in the electrocardiogram storage unit 125 and outputs the electrocardiogram to outside. The output unit 150 in this case may be a communication unit which communicates with an external device via a network. In this case, as shown in FIG. 3(A), the biological information detection device 100 is connected to an external device (for example, a server system 200) via a network NE and transmits electrocardiogram measurement result information to the server system 200. However, the external device in this case is not limited to the server system 200 and may be a smartphone or the like used by the user. Also, the network NE can be implemented by a WAN (wide area network), LAN (local area network) or the like, and can be wired or wireless. If the external device is a smartphone used by the user, the network NE can be implemented by short-range wireless communication.

Here, the information outputted from the output unit 150 will be described. Since an electrocardiogram is used for a definitive diagnosis of an arrhythmia, as described above, the output unit 150 outputs electrocardiogram measurement result information. However, the information to be outputted is not limited to this. Other information may be added to and outputted with electrocardiogram measurement result information.

For example, the output unit 150 may output electrocardiogram measurement result information in association with detection result information of arrhythmia based on pulse wave information.

Thus, for example, in the case where there is a period when electrocardiogram measurement result information is not properly acquired, it is possible to substitute detection result information based on pulse wave information for the information corresponding to this period, or the like. In the technique in this embodiment, when an arrhythmia is suspected on the basis of pulse wave information, the control to cause the electrocardiogram measuring unit 120 to operate properly or the control to notify the start of measurement is performed. However, in some cases, circumstances where an electrocardiogram is not measured can take place, such as where the user does not notice the notification, where the user cannot carry out a measuring operation for some reasons even though the user notices the notification, or where the contact of the electrode is insufficient though a measuring operation is carried out. In such cases, the period when measurement cannot be done may be regarded as having no information at all. However, given that detection result information is acquired from pulse wave information, by utilizing this detection result information, the occurrence of a data blank period can be restrained without increasing the processing load.

Also, the output unit 150 may output body movement information of the user acquired by a body movement sensor or action information of the user determined on the basis of body movement information, in association with electrocardiogram measurement result information.

In this case, the biological information detection device 100 may include a body movement sensor which acquires body movement information. The body movement information can be implemented by various sensors such as an acceleration sensor, gyro sensor, and direction sensor. The body movement information in this case is information acquired by the body movement sensor and indicating the degree of movement of the user's body. An example may be information indicating the magnitude of acceleration.

Meanwhile, the action information is information found on the basis of body movement information and indicating a specific action of the user. Various specific examples of action movement are conceivable. For example, this may be information indicating whether the user is in an awake state or in a sleeping state. In this case, the awake state may be subdivided into an exercising state and resting state. Moreover, the exercising state can be subdivided into a walking state, a running state and the like. The resting state can be subdivided into a sitting state, lying state, standing state and the like. Various methods for finding action information from body movement information are known. These methods can be broadly applied in the embodiment and therefore will not be described further in detail.

Also, the information obtained by associating body movement information (action information) with electrocardiogram measurement result information may be, for example, information obtained by associating the electrocardiogram measurement result information acquired at particular time with the body movement information acquired at the corresponding time (in a narrow sense, the same time).

In this way, not only the simple information of the electrocardiogram but also what movement (action) the user is making at the time can be outputted. There are various types of arrhythmias, and treatment strategies differ depending on the types. For example, it is known that atrial fibrillation includes sympathetic nerve-dependent atrial fibrillation and vagus nerve-dependent atrial fibrillation, and that beta blockers are effective for the former, whereas anticholineric agents are effective for the latter. It is also known that sympathetic nerve-dependent atrial fibrillation tends to occur at the time of exercise, whereas vagus nerve-dependent atrial fibrillation tends to occur at the time of rest. That is, it can be said that outputting body movement information and action information together is useful for deciding the diagnosis of arrhythmia and treatment strategies.

Also, considering that vagus nerves as the autonomic nervous system function principally as parasympathetic nerves, it is considered useful to acquire information about the activity state of the autonomic nerves of the user in terms of facilitating the classification of the sympathetic nerve-dependent atrial fibrillation and vagus nerve-dependent atrial fibrillation. Therefore, the output unit 150 may output autonomic nerve activity information acquired on the basis of pulse wave information, in association with electrocardiogram measurement result information.

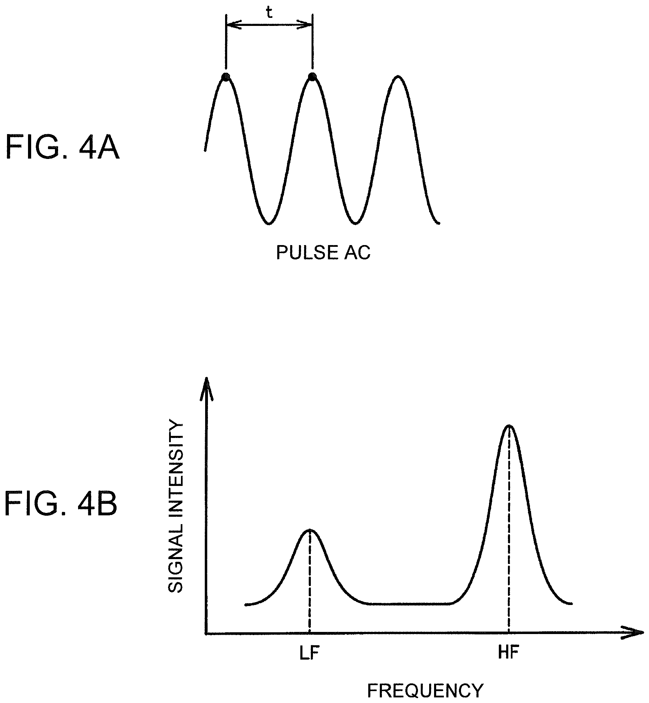

In order to determine the autonomic nerve activity state from pulse wave information, first, time-series data of the pulse interval is acquired by measuring the pulse interval indicated by t in FIG. 4(A) over a certain period. The pulse interval is not always constant but varies (fluctuates). Then, it is known that this variation is generated by the activity of sympathetic nerves and the activity of parasympathetic nerves, and it is also known that the degree of variation due to the activity of sympathetic nerves and the degree of variation due to the activity of parasympathetic nerves are different.

Thus, the time-series data of the pulse interval is frequency-converted. An example of the frequency-converted data is shown in FIG. 4(B). As can be seen from FIG. 4(B), a relatively low frequency peak LF and a relatively high frequency peak HF are acquired from the frequency-converted data.

LF indicates a slow change in the pulse interval and mainly reflects the activity of sympathetic nerves. On the other hand, HF indicates a quick change in the pulse interval and mainly reflects the activity of parasympathetic nerves. Strictly, LF reflects both sympathetic nerves and parasympathetic nerves. However, LF is described below as mainly reflecting the activity of sympathetic nerves in order to simplify the explanation.

Considering such characteristics, it is possible determine whether sympathetic nerves are predominant or parasympathetic nerves are predominant in the period of measurement of pulse wave information by finding the ratio of LF to HF (for example, the ratio of signal intensity at their respective peaks).

As autonomic nerve activity information, for example, the two values of HF and LF/HF may be used. If HF is large, it can be determined that parasympathetic nerves are predominant. If LF/HF is large, it can be determined that sympathetic nerves are predominant. The association between autonomic nerve activity information and electrocardiogram measurement result information may be carried out by associating the information acquired at the same timing, for example, as in the example of the above body movement information.

### 2.2 Example of Shape of Biological Information Detection Device

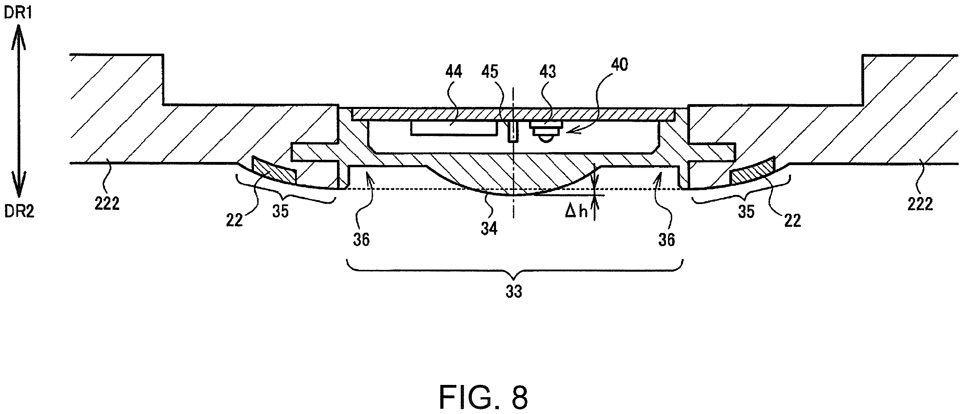

FIG. 5 shows an example of an external view of the biological information detection device 100 according to the embodiment. The biological information detection device 100 of the embodiment has a band section 10 and a case section 30, and a sensor unit 40 not shown in FIG. 5. The case section 30 is attached to the band section 10. The sensor unit 40 is provided in the case section 30, as will be described later using FIG. 8.

The band section 10 is to be wound around the user' s wrist so as to install the biological information detection device 100. The band section 10 has a band hole 12 and a buckle section 14. The buckle section 14 has a band insertion part 15 and a protruding part 16. The user inserts one end side of the band section 10 into the band insertion part 15 of the buckle section 14 and inserts the protruding part 16 of the buckle section 14 into the band hole 12 , thereby wearing the biological information detection device 100 on the wrist.

The case section 30 is equivalent to the main body part of the biological information detection device 100. Inside the case section 30, various components of the biological information detection device 100 such as the sensor unit 40 and a circuit board, not illustrated, are provided. That is, the case section 30 is a casing accommodating these components.

The biological information detection device 100 is mounted on the wrist of the user as shown in FIG. 3 (A) or the like, and the measurement of pulse wave information (in a broad sense, biological information) is carried out in this mounted state.

Also, the biological information detection device 100 includes the case section 30, which accommodates the pulse wave information measuring unit 110, the electrocardiogram measuring unit 120, and the control unit 130, as described above. The second electrode 22 for electrocardiogram measurement may be provided on the surface on the subject side of the case section 30, and the first electrode 21 for electrocardiogram measurement may be provided on the surface opposite to the surface in contact with the subject of the case section 30. Here, since the case section 30 is considered to be a hollow member accommodating various components, both inner and outer surfaces are conceivable as the surfaces of the case section 30. In such a case, considering that the first and second electrodes need to be in contact with a predetermined part of the user, the first and second electrodes should be both provided on outer surfaces of the case section 30. In short, in a narrow sense, the second electrode 22 for electrocardiogram measurement may be provided on the surface on the subject side, of the outer surfaces of the case section 30, and the first electrode 21 for electrocardiogram measurement may be provided on the surface opposite to the subject, of the outer surfaces of the case section 30.

Also, the case section 30 may have a top case 31 and a bottom case 32. In the example of FIG. 5, the case section 30 is formed by connecting the top case 31 and the bottom case 32, and the pulse wave information measuring unit 110 (in a narrow sense, the circuit board on which the pulse wave detection circuit 113 or the like is mounted) or the like is accommodated between the top case 31 and the bottom case 32. Here, the bottom case may be a plate-like (lid-like) member. In such a case, the bottom case is a back lid of the case section 30.

In this case, the second electrode 22 for electrocardiogram measurement is provided on the surface on the subject side of the bottom case 32, and the first electrode 21 for electrocardiogram measurement is provided on the side opposite to the subject of the top case 31.

FIG. 5, described above, is a plan view in the case where, if the direction from the subject (user's wrist) toward the case section 30 is defines as a first direction DR1 and the opposite direction is defined as a second direction DR2 in the state where the biological information detection device 100 is worn by the user, the biological information detection device 100 is observed from a position further toward DR1 than the biological information detection device 100.

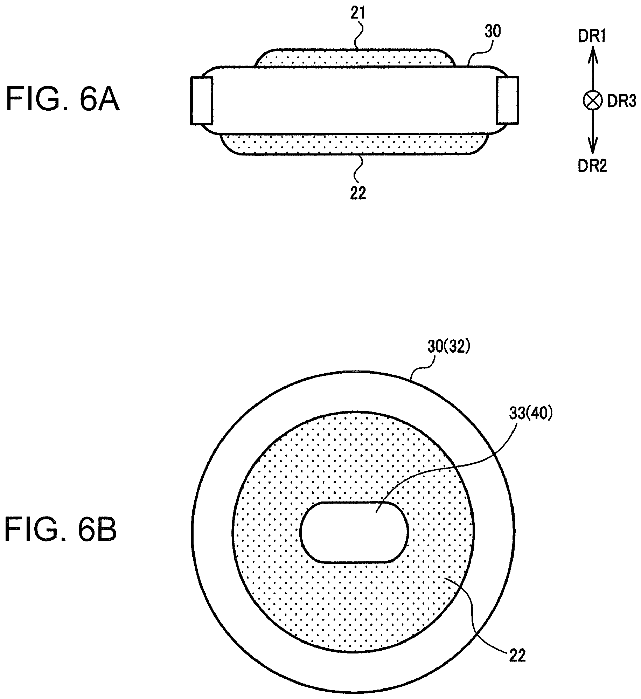

Meanwhile, FIG. 6(A) is a side view in the case where the case section 30 of the biological information detection device 100 is observed in a DR3 direction shown in FIG. 5, of the directions orthogonal to DR1 and DR2. FIG. 6 (B) is a plan view in the case where the case section 30 is observed from a position further toward DR2 than the biological information detection device 100. In this case, the case section 30 shown in FIG. 5 represents the top case 31 side. The case section shown in FIG. 6(B) represents the bottom case 32 side. Also, in FIG. 6(A), the top side on the sheet (DR1 side) is the top case 31, and the bottom side on the sheet (DR2 side) is the bottom case 32.

In this case, since the biological information detection device 100 is fixed to one wrist of the user with the band section 10, it is possible to bring the electrode section 20 in contact with the subject by providing one electrode of the electrode section 20 on the surface on the subject side of the bottom case 32 or on the surface in contact with the subject of the band section 10 (the surface on the back side of the surface illustrated in the band section 10 of FIG. 5). In this case, considering the connection between the electrode section 20 and the electrocardiogram measuring unit 120, it is desirable that the electrode section 20 (here, the second electrode 22) is provided on the surface on the subject side of the bottom case 32.

If the second electrode 22 is in contact with the wrist (for example, the left wrist) on the side where the device is mounted, the first electrode 21 needs to be insulated from the second electrode 22 and not in contact with the left wrist of the subject. This is because, in the measurement of an electrocardiogram, electrodes are arranged on the opposite sides with reference to the heart (on both sides of the heart) . To satisfy such a condition, and considering the connection between the first electrode 21 and the electrocardiogram measuring unit 120, it is desirable that the first electrode 21 is provided on the surface opposite to the subject of the top case 31.

Then, in the measurement of an electrocardiogram, the part opposite to the second electrode 22 with reference to the heart, and the first electrode 21, may be brought in contact with each other. If the biological information detection device 100 is mounted on the left arm, the first electrode 21 needs to be brought in contact with a predetermined part on the right side of the user' s body. As an example, an operation of holding the top case 31 with a right finger 1 (or the right hand) of the user may be carried out, as shown in FIG. 7(A). In this way, the first electrode 21 comes in contact with the right finger 1 (or the right hand) of the user, and a left wrist 2 and the second electrode 22 come in contact with each other, as shown in FIG. 7(B). Therefore, an electrocardiogram called lead I electrocardiogram can be measured.

Next, an example of the detailed structure of the sensor unit 40 including the pulse wave sensor 42, and the peripheral parts of the sensor unit 40, will be described. The pulse wave sensor 42 in this case may be specifically a photoelectric sensor, as described above. Light with a predetermined wavelength is observed by hemoglobin in the blood, and the amount of absorption is related to the amount of blood flow in the blood vessel. Then, the amount of blood flow periodically varies according to the beating (pulsation) of the heart. Therefore, by casing light from the light emitting unit onto the subject and receiving the reflected light thereof (transmitted light, depending on the configuration of the device), information about the amount of blood flow can be acquired and this information represents information about the pulse rate (heartbeat, pulse wave) of the subject. It is common to use a photoelectric sensor as the pulse wave sensor 42, and therefore further explanation thereof will not be given.

In this case, the light receiving unit 44 forming the pulse wave sensor should not receive all light. Specifically, the light receiving unit 44 should receive reflected light cast from the light emitting unit 43 and reflected by the subject, and should restrain reception of other light such as external disturbance light. Here, as the external disturbance light, for example, ambient light such as sunlight or illumination light is conceivable.

Therefore, the biological information detection device 100 may have a light emitting unit 43 which casts light on the subject, a light receiving unit 44 which receives the light from the subject, and a detection window 33 for casting the light from the light emitting unit 43 onto the subject and receiving the light from the subject. As shown in FIG. 2 and the like, the pulse wave information measuring unit 110 measures pulse wave information on the basis of a signal from the light receiving unit 44. The light emitting unit 43 and the light receiving unit 44 in this case form the pulse wave sensor 42. Then, the detection window 33 is provided between the pulse wave sensor 42 and the subject when the biological information detection device 100 is worn, and is provided on the surface on the subject side of the bottom case 32, as shown in FIG. 6(B), for example. The detection window 33 is formed by a light transmitting member which transmits light.

When the biological information detection device 100 is worn, the surface on the subject side of the bottom case 32 is pressed against the subject due to the load applied by the band section 10. Therefore, by placing the detection window 33 at the position shown in FIG. 6(B), external disturbance light such as ambient light can be restrained from becoming incident on the light receiving unit 44 and the detection of pulse wave information with high accuracy becomes possible. Also, in view of restraining the incidence of external disturbance light, it is desirable that the parts other than the detection window, of the surface on the subject side of the bottom case 32, is formed by a light shielding member which does not transmit light.

That is, it is desirable that the incidence of light on the case section 30 from the parts other than the detection window 33, of the case section 30 (in a narrow sense, the bottom case 32), is restrained. In this sense, the detection window 33 may be a part formed by a light transmitting member, of the part (bottom case 32) on the subject side of the case section 30.

Moreover, even if a predetermined part of the bottom case 32 is formed by a light transmitting member, if the light transmitting member is covered with a light shielding member, the incidence of light from the part of the light transmitting member can be restrained. Here, that light transmitting member is covered with the light shielding member means that the light shielding member is provided at a position further toward the subject than the light transmitting member when the device is mounted. That is, the detection window 33 may be a part made up of the exposed light transmitting member, of the part (bottom case 32) on the subject side of the case section 30. The term "exposed" here means the state where the inside of the case section 30 can be observed from outside in the circumstance where the biological information detection device 100 is used in a normally expected form (without dismantling or the like). As an example, being observable in the case where the bottom case 32 is observed from a point of view set at a position further toward DR2 than the bottom case 32, as shown in FIG. 6(B), can be defined as being "exposed".

As described above, one of the electrodes (second electrode 22) of the electrode section 20 may be provided on the surface on the subject side of the case section 30. However, since an electrode is generally made of a metal or the like which does not transmit light, it is not desirable that the electrode overlaps with the position of the detection window 33. Conversely, it can be said that the overlapping of the light shielding member and the electrode overlap, or the use of the electrode as the light shielding member is desirable.

That is, the electrode section 20 for measuring an electrocardiogram (in a narrow sense, one electrode of the electrode section, and the second electrode 22 in this case) may be provided in a peripheral part of the detection window 33. In the example of FIG. 6(B), the second electrode 22 is provided in such a way as to surround the detection window 33, as viewed in a plan view where the case section 30 is observed from a point of view set further toward DR2 than the case section 30. However, the second electrode 22 is not limited to the shape of concentric circles shown in FIG. 6(B) with the detection window 33 arranged inside the inner circle of the concentric circles. For example, an electrode in a shape similar the first electrode 21 shown in FIG. 5 (elliptic shape as viewed in a plan view) may be arranged near the detection window 33 and used as the second electrode 22.

FIG. 8 shows the cross-sectional structure of the sensor unit 40, the detection window 33 and its peripheries. FIG. 8 is a cross-sectional view in the case where the sensor unit 40 or the like is observed in the direction of DR3, as in FIG. 6(A), for example. As shown in FIG. 8, the sensor unit 40 includes the light emitting unit 43 and the light receiving unit 44. The pulse wave sensor 42 is realized by the light emitting unit 43 and the light receiving unit 44. Also, direct light from the light emitting unit 43 to the light receiving unit 44 includes no components relating to pulse waves, that is, this light is a noise component. Therefore, the sensor unit 40 may also include a light shielding wall 45 which blocks the incidence of the direct light on the light receiving unit 44.

Also, the detection window 33 may have a protruding part 34 to apply a pushing pressure to the subject, and the biological information detection device 100 (particularly the case section 30, and in a narrow sense, the bottom case 32) may further include a pushing pressure restraining part 35 which restrains the pushing pressure applied to the subject by the protruding part 34.

Then, in the embodiment, the protruding part 34 protrudes toward the subj ect from a pushing pressure restraining surface of the pushing pressure restraining part 35 in such a way as to achieve Δh>0. That is, the protruding part 34 protrudes toward the subj ect by the amount of Δh from the pushing pressure restraining surface of the pushing pressure restraining part 35.

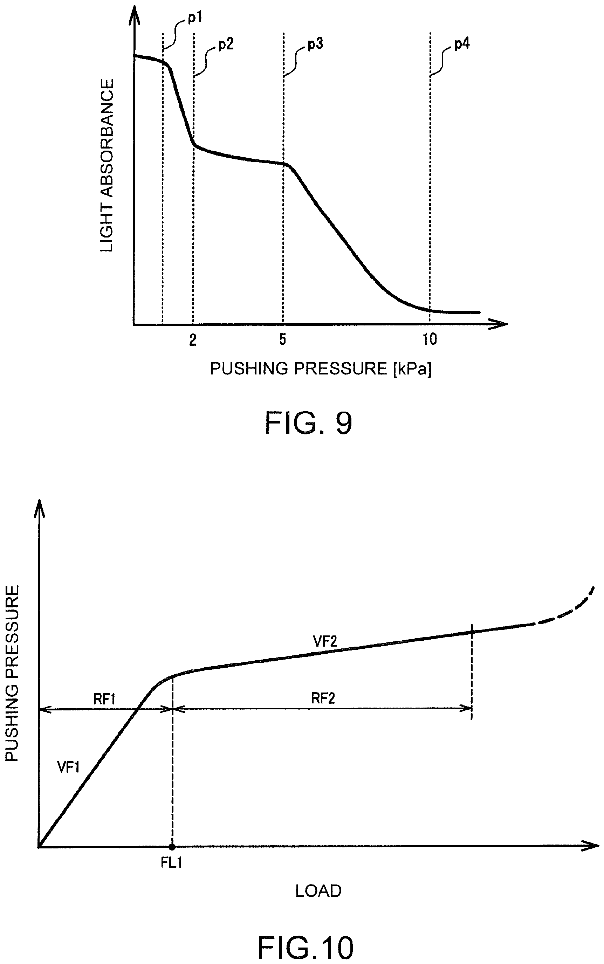

By thus providing the protruding part 34 to achieve Δh>0, it is possible to apply to the subject, for example, an initial pushing pressure for exceeding a vein vanishing point. Also, by providing the pushing pressure restraining part 35 for restraining the pushing pressure applied to the subject by the protruding part 34, it is possible to minimize the change in the pushing pressure within the range of use where biological information is measured by the biological information detection device 100, and therefore a reduction of noise components or the like can be achieved. Also, if the protruding part 34 protrudes from the pushing pressure restraining surface in such a way as to achieve Δh>0, after the protruding part 34 comes in contact with the subject and applies the initial pushing pressure, the pushing pressure restraining surface of the pushing pressure restraining part 35 comes in contact with the subject and can restrain the pushing pressure applied to the subj ect by the protruding part 34. Here, the vein vanishing point refers to a point where a signal due to the vein superimposed on a pulse wave signal vanishes or decreases to a level that does not affect pulse wave measurement, when the protruding part 34 is brought in contact with the subject and the pushing pressure is gradually increased. The vein vanishing point is equivalent to p2 in FIG. 9.

FIG. 9 is a view illustrating a change in light absorbance with respect to pushing pressure. The horizontal axis represents pushing pressure and the vertical axis represents light absorbance in the blood vessel. As the pushing pressure changes, the affected blood vessel changes. The most susceptible blood vessel, that is, the blood vessel that is affected by the lowest pushing pressure, is the capillary. In the example of FIG. 9, the amount of change in light absorbance increases at the point where the pushing pressure exceeds p1. This means that the capillary begins to be flattened by the pushing pressure. As the pushing pressure exceeds p2, the change in light absorbance becomes gentle. This means that the capillary is flattened (closed) almost completely. The next most susceptible part following the capillary is the artery. As the pushing pressure increases further and exceeds p3, the amount of change in light absorbance increases again. This means that the artery begins to be flattened by the pushing pressure. As the pushing pressure exceeds p4, the change in light absorbance becomes gentle. This means that the artery is flattened (closed) almost completely.

For example, in FIG. 10, the horizontal axis represents the load generated by the band section 10 or the like, and the vertical axis represents the pushing pressure (pressure applied to the blood vessel) applied to the subject by the protruding part 34. Then, it is assumed that the amount of change in the pushing pressure of the protruding part 34 with respect to the load by a load mechanism which generates the pushing pressure of the protruding part 34 is referred to as the amount of pushing pressure change. The amount of pushing pressure change is equivalent to the gradient of the pushing pressure change characteristic with respect to the load.

In this case, the pushing pressure restraining part 35 restrains the pushing pressure applied to the subject by the protruding part 34 in such a way that, compared with an amount of pushing pressure change VF1 in a first load range RF1 where the load of the load mechanism is 0 to FL1, an amount of pushing pressure change VF2 in a second load range RF2 where the load of the load mechanism is greater than FL1 is smaller. That is, while the amount of pushing pressure change VF1 is increased in the first load range RF1, which is an initial pushing pressure range, the amount of pushing pressure change VF2 is decreased in the second load range RF2, which is the range of use of the biological information detection device 100.

That is, in the first load range RF1, the amount of pushing pressure change VF1 is increased and the gradient of the pushing pressure change characteristics with respect to the load is thus increased. Such a pushing pressure with a large gradient of the change characteristic is realized by Δh equivalent to the amount of protrusion of the protruding part 34. That is, by providing the protruding part 34 which achieved Δh>0, it is possible to apply to the subject a necessary and sufficient initial pushing pressure to exceed the vein vanishing point even in the case where the load by the load mechanism is small.

Meanwhile, in the second load range RF2, the amount of pushing pressure change VF2 is decreased and the gradient of the pushing pressure change characteristic with respect to the load is thus decreased. Such a pushing pressure with a small gradient of the change characteristic is realized by the restraining of the pushing pressure by the pushing pressure restraining part 35. That is, as the pushing pressure restraining part 35 restrains the pushing pressure applied to the subject by the protruding part 34, it is possible to minimize the variation in the pushing pressure even in the case where there is some variation in the load or the like, in the range of use of the biological information detection device 100. Thus, a reduction of noise components or the like can be achieved.

By thus causing the optimized pushing pressure (approximately 16 kPa, for example) to be applied to the subject, it is possible to obtain a pulse wave detection with a higher M/N ratio (S/N ratio). That is, the signal component of the pulse sensor 42 can be increased and noise components can be reduced. Here, M indicates the signal level of a pulse wave detection signal, and N indicates the noise level.

Also, a groove part 36 may be provided in the detection window 33, as shown in FIG. 8. The height of the bottom surface of the groove part 36 is lower than the height (height of the highest end part) of the outer surface (surface on the subject side at the time of mounting) of the pushing pressure restraining part 35. The bottom surface of the groove part 36 is a surface below the outer surface (toward the sensor unit 40).

For example, if a relatively soft thing such as skin is brought in contact with a contact surface of a light transmitting member of a hard material made of a resin, an area which is not in contact with the skin or an area with a low contact pressure is generated near peripheral edge part (outer peripheral part) of the light transmitting member. Therefore, for example, if the perimeter of the protruding part 34 is not provided with the groove part 36 and is made as a flat part, this flat part may not come in contact with the skin or may result in a weak contact state, thus causing the contact state to change dynamically. Then, due to such dynamic change of the contact state, variation in the optical intensity of light tends to occur. If such light becomes incident on the light receiving unit, it results in a noise having no correlation with the pulse component.

In this respect, if the groove part 36 as shown in FIG. 8 is provided, the generation of an area where the contact state dynamically changes in this way can be prevented effectively and therefore improvement in signal integrity or the like can be achieved.

In this case, it is preferable that one (second electrode 22) of the electrodes for measuring an electrocardiogram is provided in the pushing pressure restraining part 35. As described above, because of its characteristic of controlling the pushing pressure on the subject at the time of measurement, the pushing pressure restraining part 35 tends to be in the state of tight contact with the subject. Therefore, by providing the electrode in the pushing pressure restraining part 35, it is possible to bring the subject and the electrode firmly in contact with each other and therefore to securely carry out the measurement of an electrocardiogram, or the like. As an example, as shown in FIG. 8, a surface part of the pushing pressure restraining part 35 (while it is a part of the surface in FIG. 8, it may be the entire surface) may be realized by the second electrode 22. Alternatively, the second electrode 22 may be provided in such a way as to protrude from the pushing pressure restraining part 35, if it increases the degree of contact between the electrode section 20 and the subject.

### 2.3 Example of System Including Biological Information Detection Device

In the example of FIG. 2, it is assumed that the analysis processing unit 140 performing arrhythmia analysis processing on the basis of pulse wave information is included in the biological information detection device 100. However, the configuration in the embodiment is not limited to this and the configuration shown in FIG. 11 may be employed as well. In FIG. 11 the biological information detection device 100 includes a receiving unit 170 which receives detection result information of arrhythmia on the basis of pulse wave information. The control unit 130 performs at least one of the power supply control, the storage control, and the notification control on the basis of the received detection result information.

In this case, the output unit 150 of the biological information detection device 100 outputs the pulse wave information read out from the pulse wave information storage unit 115 to another information processing device, and the arrhythmia analysis processing based on the pulse wave information is carried out by this another information processing device. In the biological information detection device 100, the result of the analysis processing is received by the receiving unit 170. The subsequent control by the control unit 130 is similar to the configuration of FIG. 2. FIG. 2 and FIG. 11 differ from each other in terms of whether the analysis processing is carried out inside the biological information detection device 100 or in the external device.

In the case of the configuration of FIG. 11, it is possible to use the server system 200 as an information processing device by employing a configuration similar to the example shown in FIG. 3 (A). Since the biological information detection device 100 worn by the user needs to be small-sized and lightweight, there is a great restriction on the battery, the processing capability of the processing unit inside the device, or the data storage capacity. Meanwhile, the server system 200 has a relatively small restriction on its resources and therefore can perform the arrhythmia analysis processing or the like at a high speed and hold a greater amount of data (pulse wave information or arrhythmia analysis result).

The server system 200 in this case may be the same device as the device as the output destination of electrocardiogram measurement result information, or may be a different device. Also, the above information processing device may be realized by a processing device (in a narrow sense, a mobile terminal device) such as a smartphone 300, instead of the server system 200. An example of the configuration in this case is FIG. 3 (B) . The mobile terminal device such as the smartphone 300 has more restrictions than the server system 200 on the processing capability, the storage area, and the battery capacity. However, considering the recent improvements in performance, it is conceivable that sufficient performance capability or the like can be secured. Therefore, if the requirements of processing capability and the like are satisfied, a smartphone or the like can be used the above-described another information processing device, as shown in FIG. 3(B).

Also, in the case where the biological information detection device 100 according to the embodiment and the server system 200 are connected to each other, these may be directly connected via the network NE as shown in FIG. 3(A) or may be connected via another device such as a smartphone as shown in FIG. 3(C). The form of connection can be implemented with various modifications. Also, the server system 200 in this case may be a device which performs arrhythmia analysis processing as described above, a device as the output destination of electrocardiogram measurement result information, or a device functioning as both.

### 3. Example of Control Using Detection Result Information of Arrhythmia

Next, an example of the controls performed by the control unit 130 will be described. The control unit 130 performs the power supply control to start supplying electric power to the electrocardiogram measuring unit 120 if it is determined that an arrhythmia is detected on the basis of pulse wave information.

Specifically, the control to start supplying electric power to the electrocardiogram measuring unit 120 (in a narrow sense, the electrocardiogram detection circuit 123) from a battery not illustrated in FIG. 2 or the like may be performed. As described above, the case where an arrhythmia is detected is the case where, as an electrocardiogram is measured, it is determined that there is a great need to output the measured electrocardiogram. Thus, by performing the power supply control to start supplying electric power, it is possible to measure an electrocardiogram by the electrocardiogram measuring unit 120 and therefore it is possible to acquire an electrocardiogram at an appropriate timing (period).

Also, this power supply control can be regarded as the control not to supply electric power to the electrocardiogram measuring unit 120 or the control to regulate the power supply, if it is not determined that an arrhythmia is detected on the basis of pulse wave information. That is, by performing such power supply control, it is possible not to supply electric power or to reduce power consumption in the circumstance where the need to measure an electrocardiogram is low.

However, even if an arrhythmia is not detected on the basis of pulse wave information, there can be cases where the user wishes to measure an electrocardiogram on his/her own decision. Therefore, instead of performing the power supply control simply on the basis of the arrhythmia detection result based on pulse wave information, other information may also be used together. As an example, electric power may be supplied to the electrocardiogram measuring unit 120 if an arrhythmia is detected on the basis of pulse wave information, or if an explicit measurement instruction is given by the user (for example, the operation unit is operated), whereas electric power is not supplied otherwise.

Also, the control unit 130 may perform the storage control to start storing electrocardiogram measurement result information in the storage unit (electrocardiogram storage unit 125) if it is determined that an arrhythmia is detected on the basis of pulse wave information.

For the viewer (user operating the output destination device, for example, a doctor) to make a certain determination on the basis of electrocardiogram measurement result information, it is desirable that this electrocardiogram measurement result information is not data at a timing or over a short span such as a few seconds but is time-series data continuing for approximately a few hours, for example. That is, it is assumed that the measurement of an electrocardiogram by the electrocardiogram measuring unit 120 is carried out continuously to a certain extent.

In such a case, if the measured information is to be outputted successively from the output unit 150, the storage of electrocardiogram measurement result information inside the biological information detection device 100 need not be considered so much. However, considering the power consumption or the like due to output processing (communication processing), a certain amount of data may be accumulated and stored and the output may be carried out using the accumulated data as a unit.

Considering the above points, since the storage of electrocardiogram measurement result information, too, is an important element in the measurement of an electrocardiogram, the control unit 130 may perform the storage control. As described above, what is to be stored is the electrocardiogram measurement result information corresponding to the period when the occurrence of an arrhythmia is suspected. Therefore, the storage may start when it is determined that an arrhythmia is detected on the basis of pulse wave information.

Also, if it is not determined that an arrhythmia is detected on the basis of pulse wave information, it is possible not to carry out the storage or regulate the storage. In this case, since the information stored in the electrocardiogram storage unit 125 is limited to the information corresponding to the period when the occurrence of an arrhythmia is suspected, information estimated as having little relation to arrhythmia, that is, information with low priority, can be restrained from being stored in the electrocardiogram storage unit 125 or from being outputted via the output unit 150. However, as in the above example of the power supply control, the storage control may be carried out using information other than the arrhythmia detection result based on pulse wave information.

Also, the control unit 130 may perform the notification control to start outputting (in a narrow sense, outputting to the user) notification information to prompt the measurement of an electrocardiogram using the electrocardiogram measuring unit 120 if it is determined that an arrhythmia is detected on the basis of pulse wave information.

As shown in FIG. 5 or the like, in the biological information detection device 100 according to the embodiment, the electrode section 20 used to measure an electrocardiogram is not necessarily always in contact with the measurement site of the subject. In the case of the biological information detection device 100 of FIG. 5, it is necessary to make the user carry out the action of contacting the first electrode 21 with his/her right finger (right hand), as described above with reference to FIGS. 7(A) and 7(B).

In this way, in the case of the embodiment where the user is forced to carry out a predetermined action to measure an electrocardiogram, the recognition by the biological information detection device 100 that an arrhythmia is detected on the basis of pulse wave information is insufficient, and electrocardiogram measurement cannot be carried out unless the information to that effect is communicated to the user. Thus, in the embodiment, when it is determined that an arrhythmia is detected on the basis of pulse wave information, the user is prompted to measure an electrocardiogram. Specifically, a notification to prompt measurement is given by the notification unit 160.

If the notification unit 160 includes a display unit 161 as shown in FIG. 2, a certain display is shown on the display unit 161, thus prompting measurement. The notification in this case may be text information such as "Start measuring the electrocardiogram" or "Put your right hand on the electrode", image information showing the user posture as shown in FIG. 7 (A), or a combination of these. Alternatively, a part of the entirety of the display unit 161 may simply emit light in a predetermined color.

Also, if the notification unit 160 includes a buzzer 163 as shown in FIG. 2, a notification which generates a predetermined buzz may be used. If the notification unit 160 can output a speech, the notification unit 160 may read out a speech such as "Start measuring the electrocardiogram". Alternatively, if the notification unit 160 includes a vibrating unit, not illustrated, the notification unit 160 may cause the vibrating unit to vibrate, thus giving a notification. In addition, the specific configuration of the notification unit 160 and the contents of the notification prompting the measurement can be implemented with various modifications.

The controls on the start of the supply of electric power, the start of the storing, and the start of the notification have been described above, and various control methods for halting these are conceivable as well. For example, the control unit 130 may perform halt control on the supply of electric power, halt control on the storage of electrocardiogram measurement result information, or halt control on the output of notification information, on the basis of arrhythmia detection result information.

Specifically, when it is no longer determined that an arrhythmia is detected on the basis of pulse wave information, a halt to the power supply to the electrocardiogram measuring unit, a halt to the storage, and a halt to the notification may be performed. In this way, since the supply of electric power and the storage of electrocardiogram measurement result information area carried out during the period when it is determined that an arrhythmia is detected on the basis of pulse wave information, power consumption can be reduced and information with low priority can be restrained from being stored, as described above. Also, if the notification control to prompt measurement is continued during the period when it is determined that an arrhythmia is detected, the user can understand that he/she can end the measurement as this notification control ends.

It is desirable that the power supply control to supply electric power and the storage control to store electrocardiogram measurement result information are continued during the period when it is determined that an arrhythmia is detected. Otherwise, a period during which an electrocardiogram is not measured despite the occurrence of an arrhythmia may take place.

However, with respect to the notification control, if the user has started measuring an electrocardiogram in response to the notification control, continuing the notification will be annoying to the user. Therefore, the notification control to prompt measurement may be halted regardless of the arrhythmia detection result based on pulse wave information. For example, the notification control may be halted at a timing with the lapse of a predetermined time following the start of the notification control. Alternatively, electrocardiogram measurement result information is monitored, and the notification control may be halted at a timing when it is determined that the measurement by the user is started, that is, that the acquisition of a signal expected as a human electrocardiogram is started.

In such a case, in view of user-friendliness, at a timing when the operation for electrocardiogram measurement may be ended, a notification to that effect may be given. That is, notification control to prompt electrocardiogram measurement may be performed, and the notification control may be halted with the lapse of a predetermined time or the like. Subsequently, notification control to prompt the end of the electrocardiogram measurement may be performed separately. As an example, when it is no longer determined that an arrhythmia is detected on the basis of pulse wave information, the notification control to prompt the end of the electrocardiogram measurement may be performed. Also, with the lapse of a predetermined period following the detection of an arrhythmia based on pulse wave information, the notification control to prompt the end of the electrocardiogram measurement may be performed. Moreover, control may be performed in such a way as to start electrocardiogram measurement and also halt at least a part of the functions of the pulse wave information measuring unit 110 after the detection of an arrhythmia based on pulse wave information, then to carry out the detection of pulse wave information by the pulse wave information measuring unit 110 when a predetermined period has passed following the start of the electrocardiogram measurement, and to end the electrocardiogram measurement when it is no longer determined that an arrhythmia is detected on the basis of pulse wave information. In this case, since supplying electric power to both the electrocardiogram measuring unit and the pulse wave information measuring unit can be avoided, power consumption can be reduced further.

Also, in the biological information detection device 100 according to the embodiment, all of the above power supply control, storage control, and notification control may be performed. However, this is not limiting and modifications can be made such as performing one of these or selectively combining two of them.

### 4. Arrhythmia Detection Technique

A technique for detecting an arrhythmia on the basis of pulse wave information will be described. The processing described here is carried out by the analysis processing unit 140 of the biological information detection device 100 or by a processing unit of a processing device other than the biological information detection device 100. Hereinafter, an example carried out by the analysis processing unit 140 is described. Also, arrhythmia is divided into atrial fibrillation, tachyarrhythmia, and bradyarrhythmia herein and their respective detection methods are described. Strictly speaking, atrial fibrillation is tachyarrhythmia. However, considering that the importance of detecting atrial fibrillation is high since it causes critical conditions such as cerebral infarction and that not only the simple pulse rate but also the irregularity of the pulse interval is used for the detection, atrial fibrillation is explained separately from tachyarrhythmia.

### 4.1 Atrial Fibrillation

The control unit 130 of the biological information detection device 100 according to the embodiment may perform at least one of power supply control, storage control, and notification control, using the result of detection of atrial fibrillation as arrhythmia detection result information. Atrial fibrillation is determined on the basis of the irregularity of the pulse interval (RR interval).

However, in the case of measuring pulse waves, the subject can often move around freely during measurement and therefore the influence of body movement noise tends to be included in the pulse wave signal. In the case of measuring an electrocardiogram, too, the influence of body movement noise maybe included in the waveform signal of the electrocardiogram, though to a different degree from the case of measuring pulse waves. In the case where the measurement is thus influenced by body movement noise, it is very difficult to accurately measure the RR interval per beat. In the embodiment, a technique for restraining the influence of body movement noise and detecting atrial fibrillation with high accuracy even when pulse wave information is used, is used.

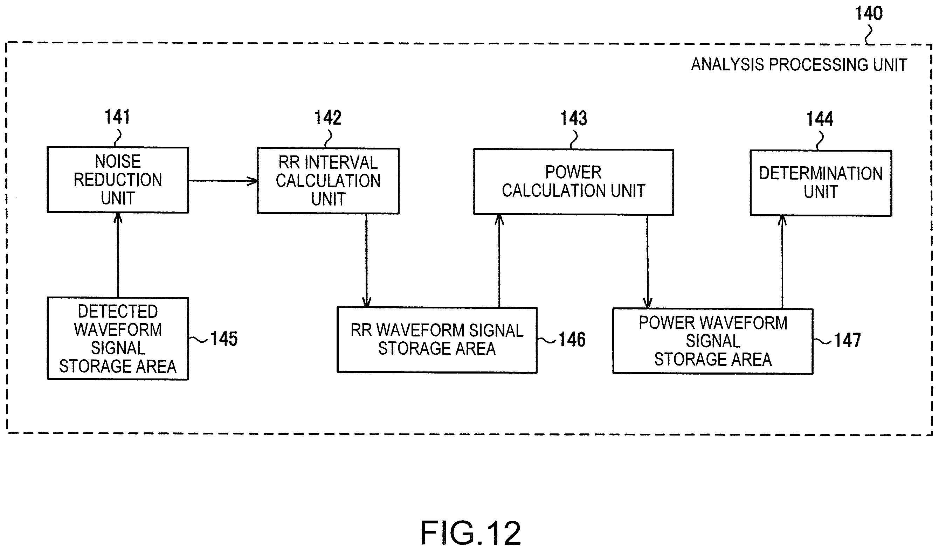

The analysis processing unit 140 has a noise reduction unit 141, an RR interval calculation unit 142, a power calculation unit 143, and a determination unit 144, and is also implemented by the functional configurations of a detected waveform signal storage area 145, an RR waveform signal storage area 146, and a power waveform signal storage area 147, which are storage areas for various data, as shown in FIG. 12.

The noise reduction unit 141 performs filter processing to reduce a body movement noise component other than a frequency band corresponding to the RR interval, in a detected waveform signal L stored in the detected waveform signal storage area 145, and then outputs the signal. The detected waveform signal storage area 145 and the noise reduction unit 141 function as an acquisition unit which acquires the detected waveform signal L used for frequency analysis by the RR interval calculation unit 142.

Meanwhile, in this processing, while the body movement noise component is reduced and its influence is reduced in the detected waveform signal L, it falls short of enabling measurement of an RR interval that is accurate enough to enable determination of atrial fibrillation to the same extent as in the case of using an electrocardiogram.

The RR interval calculation unit 142 slices out a frame for each sampling from the detected waveform signal L in which the body movement noise component is reduced by the noise reduction unit 141, and calculates a frequency spectrum by frequency analysis over a short period of time (STFT (short-time Fourier transform) analysis). Then, the RR interval calculation unit 142 calculates a parameter corresponding to the RR interval for each frame on the basis of the calculated frequency spectrum, and stores an RR waveform signal FRR indicating a change with time of this parameter, in the RR waveform signal storage area 146.

In this example, the calculated parameter is a value indicating the average of RR intervals in the frame, and for example, a maximum peak frequency on the frequency spectrum. Therefore, the RR waveform signal FRR indicates a change with time of the average pulse wave RR interval. With the processing by the RR interval calculation unit 142, the influence of the body movement noise included in the RR waveform signal FRR can be reduced significantly even if the body movement noise cannot be completely eliminated by the noise reduction unit 141.

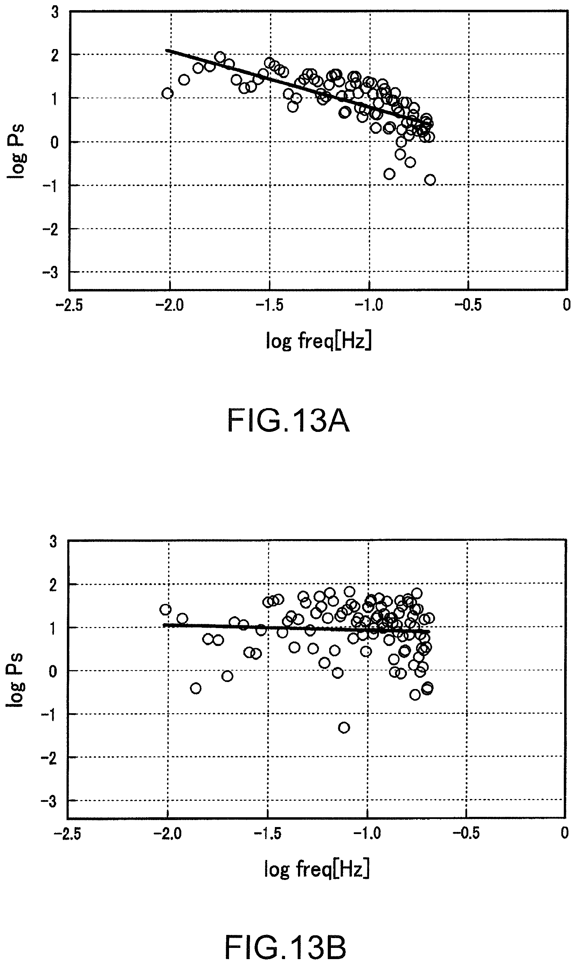

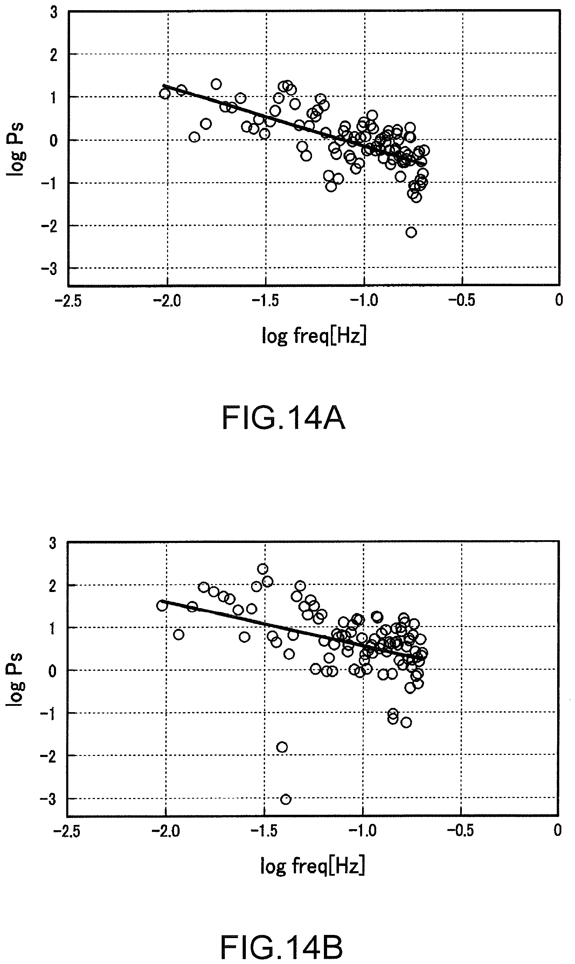

FIG. 13(A) and FIG. 13(B) are graphs showing logarithmically converted values of peak frequency and power, obtained by frequency analysis in a band of 0.01 Hz to 0.2 Hz on a frame of 480 seconds of a waveform signal indicating variation in the electrocardiogram RR interval. FIG. 14(A) and FIG. 14(B) are graphs in the case where similar frequency analysis is carried out on the output of RR interval calculation unit 142, that is, on the average pulse wave RR interval. FIG. 13(A) and FIG. 14(A) are graphs in the case where atrial fibrillation has not occurred. FIG. 13 (B) and FIG. 14 (B) are graphs in the case where atrial fibrillation has occurred.

As can be understood from FIG. 13 (A) and FIG. 13 (B), in the case where the electrocardiogram RR interval is used, the intercept and gradient of the regression line change depending on whether atrial fibrillation has occurred or not, and therefore atrial fibrillation can be determined on the basis of the intercept and gradient. However, as can be understood from FIG. 14 (A) and FIG. 14 (B), in the case where the average pulse wave RR interval is used, though the body movement noise can be reduced, the difference in the intercept and gradient between the presence and absence of the occurrence is small and therefore similar determination is difficult.

However, in the case where focus is on the high frequency band in FIG. 14(A) and FIG. 14(B), when atrial fibrillation has occurred, the power is several times greater than when atrial fibrillation has not occurred, and therefore a significant difference is observed. Therefore, the change in power in this frequency band can be an indicator for determining whether atrial fibrillation has occurred or not. In the embodiment, the determination on atrial fibrillation is carried out on the basis of this change in power.

The power calculation unit 143 performs frequency analysis in a short time (STFT analysis) on the RR waveform signal FRR stored in the RR waveform signal storage area 146, and calculates the power (hereinafter referred to as band power) of a certain frequency band (hereinafter referred to as calculation frequency band) on the basis of the resulting frequency spectrum. The power calculation unit 143 stores a power waveform signal Pa indicating a change with time in the calculated band power, in the power waveform signal storage area 147.

The determination unit 144 determines whether a specific determination condition is satisfied or not, with respect to the power waveform signal Pa stored in the power waveform signal storage area 147, and outputs information corresponding to the result of the determination. The specific determination condition in this example means that the time during which the value of the power waveform signal Pa (band power) exceeds a predetermined threshold Pth (for example "1") is 50% or more of the observation time of 30 minutes preceding the time point of determination.

However, the specific determination condition can be set in various manners. It is possible to determine that atrial fibrillation has occurred if the value of the power waveform signal Pa exceeds the threshold Pth over a predetermined period of time. Also, it is possible to determine that atrial fibrillation has occurred if the number of times the threshold Pth is exceeded is above a predetermined number of times even if the time during which the threshold value Pth is exceeded is less than 50% of the observation time of 30 minutes. It is also possible to determine that atrial fibrillation has occurred if the integral value of the band power during the 30 minutes exceeds a predetermined value.

Also, if it is not real-time determination, an observation time of 15 minutes each preceding and following the time point of determination may be used, for example, instead of the observation time of 30 minutes preceding the time point.

In the embodiment, the time during which the power exceeds Pth, of the observation time, can be used as an AF rate (degree of reliability). That is, the determination unit 144, which outputs the result of determination on atrial fibrillation to the control unit 130 or the like, may output the information of the AF rate as well. The AF rate is outputted from the output unit 150, for example, in association with electrocardiogram measurement result information.

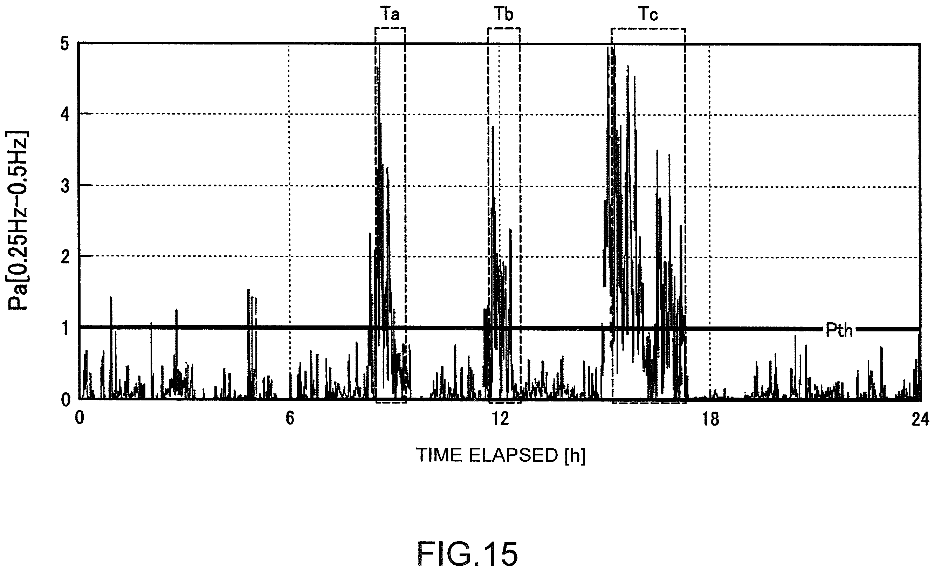

FIG. 15 is a view illustrating periods that are determined as atrial fibrillation. The waveform shown in FIG. 15 is an example of the waveform of the power waveform signal Pa and is a waveform obtained by detecting pulse waves over 24 hours. Periods Ta, Tb, Tc represent periods determined as atrial fibrillation by the determination unit 144. In these periods Ta, Tb, Tc, the value of the power waveform signal Pa is high, as shown in FIG. 15.

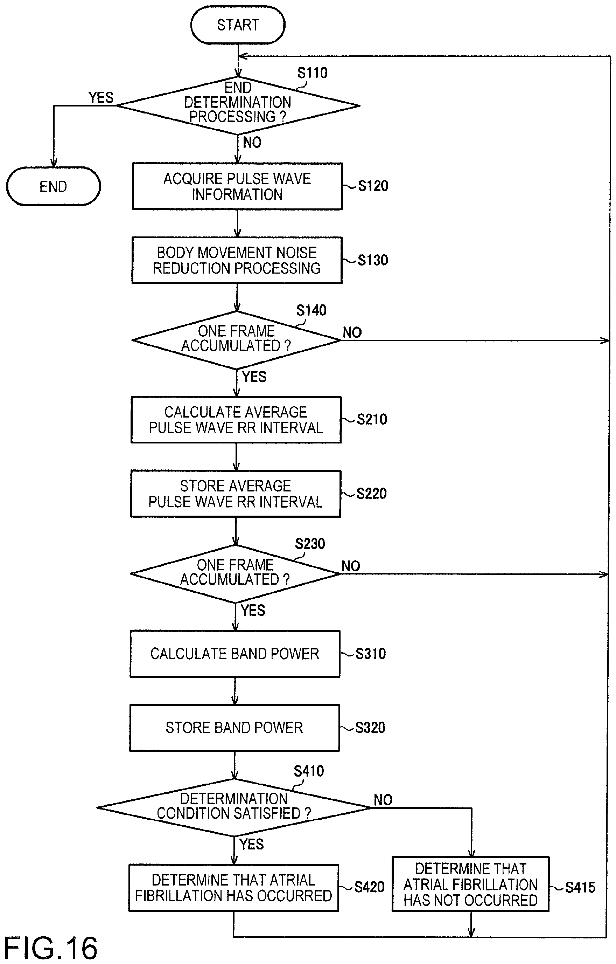

FIG. 16 is a flowchart illustrating atrial fibrillation determination processing in the embodiment. First, as an instruction to start atrial fibrillation determination processing is inputted by the user, the analysis processing unit 140 starts the flow shown in FIG. 16. The analysis processing unit 140 determines whether an instruction to end the determination processing is inputted by the user or not (Step S110)*.* If an instruction to end the determination processing is inputted (Step S110; Yes), the analysis processing unit 140 ends the atrial fibrillation determination processing.

If an instruction to end the determination processing is not inputted (Step S110; No), the analysis processing unit 140 acquires pulse wave information (detected wave signal L) from the pulse wave information measuring unit 110 (Step S120) and carries out body movement noise reduction processing by the noise reduction unit 141 (Step S130). At this time, the analysis processing unit 140 stores the detected waveform signal L in the detected waveform signal storage area 145 and may store the detected waveform signal L on which the body movement noise reduction processing has been carried out.

The analysis processing unit 140 determines whether the waveform signal on which the body movement noise reduction processing has been carried out has been accumulated by one frame in the RAM or not (Step S140). If the signal has not been accumulated by one frame (Step S140; No), the analysis processing unit 140 returns to Step S110 and continues the processing. Meanwhile, if the signal has been accumulated by one frame (Step S140; Yes), the analysis processing unit 140 calculates an average pulse wave RR interval by the RR interval calculation unit 142 (Step S210).

The analysis processing unit 140 stores the average pulse wave RR interval calculated by the RR interval calculation unit 142, in the RR waveform signal storage area 146 (Step S220). The change with time of the average pulse wave RR interval stored in this storage area is the RR waveform signal FRR.

The analysis processing unit 140 determines whether the RR waveform signal FRR stored in the RR waveform signal storage area 146 has been accumulated by one frame or not (Step S230). If the signal has not been accumulated by one frame (Step S230; No9), the analysis processing unit 140 returns to Step S110 and continues the processing. Meanwhile, if the signal has been accumulated by one frame (Step S230; Yes), the analysis processing unit 140 calculates a band power by the power calculation unit 143 (Step S310).

The analysis processing unit 140 stores the band power calculated by the power calculation unit 143, in the power waveform signal storage area 147 (Step S320). The change with time of the band power stored in this storage area is the power waveform signal Pa.

The analysis processing unit 140 determines by the determination unit 144 whether the atrial fibrillation determination condition that 50% or more of the band power during the preceding 30 minutes exceeds the threshold Pth is satisfied or not, referring to the stored power waveform signal Pan (Step S410). If it is determined that the determination condition is not satisfied (Step S410; No), the analysis processing unit 140 determines that atrial fibrillation has not occurred (Step S415), returns to Step S110, and continues the processing.

Meanwhile, if it is determined that the determination condition is satisfies (Step S410; Yes), the analysis processing unit 140 determines that atrial fibrillation has occurred (Step S420), returns to Step S110, and continues the processing.

In this way, the analysis processing unit 140 in the embodiment can carry out the determination on atrial fibrillation while reducing the influence of body movement noise by measuring the average pulse wave RR interval instead of the pulse wave RR interval per beat.

### 4.2 Tachyarrhythmia and Bradyarrhythmia

The control unit 130 may perform at least one of power supply control, storage control, and notification control, using a tachycardia detection result as arrhythmia detection result information. Also, the control unit 130 may perform at least one of power supply control, storage control, and notification control, using a bradycardia detection result as arrhythmia detection result information.

Tachycardia or bradycardia can be detected by finding the pulse rate. For example, an average value of pulse wave interval per beat for several arbitrary seconds (for example, 15 seconds) may be found from pulse wave information and converted to pulse rate per minute, thus finding the pulse rate. Also, various methods for finding the pulse rate from pulse wave information (particularly, pulse AC signal) are known and these are broadly applicable in the embodiment.

As for Tachycardia, a state where the pulse rate is abnormally high may be detected. Specifically, if it is determined that the pulse rate is equal to or above a first pulse rate threshold (for example, 150 or above) and that the user is in a resting state on the basis of body movement (or action information), this state is determined as tachycardia. Body movement information can be acquired from sensor information of a body movement sensor such as an acceleration sensor, as described above. Normally, in a resting state, the pulse rate should a relatively low numerical value. That is, if the pulse rate is relatively high (first threshold or above) despite being in a resting state, this state is an abnormal state and therefore determined as tachycardia.

Also, even if it is determined that the user is in an exercising state (activity state) on the basis of body movement information (or action information), the state is detected as tachycardia if the pulse rate is equal to or above a second pulse rate threshold (for example, 200 or above). This is because, while the pulse rate tends to be higher in the first place if the user is in an exercising state, the state can be determined as abnormal if the condition that the pulse rate is equal to or above the second pulse rate threshold, which is a more difficult condition, is satisfied. That is, the second pulse rate threshold is a higher value than the first pulse rate threshold.

In contrast, as for bradycardia, a state where the pulse rate is abnormally low may be detected. Specifically, if the pulse rate is equal to or below a third pulse rate threshold (typically 40 or below), this state is detected as bradycardia.

While the embodiment has been described in detail above, a person skilled in the art can readily understand that a number of modifications can be made without substantially departing from new matters and advantageous effects of the invention. Therefore, all such modifications are included in the scope of the invention. For example, a term described with a different term with a broader meaning or the same meaning at least once in the specification or drawings can be replaced with the different term at any point in the specification or drawings. Also, the configurations and operations of the biological information detection device are not limited to those described in the embodiment can be carried out with various modifications.

### Reference Signs List

1 right finger, 2 left wrist, 10 band section, 12 band hole, 14 buckle section, 15 band insertion part, 16 protruding part, 20 electrode section, 21 first electrode, 22 second electrode, 30 case section, 31 top case, 32 bottom case, 33 detection window, 34 protruding part, 35 pushing pressure restraining part, 36 groove part, 40 sensor unit, 42 pulse wave sensor, 43 light emitting unit, 44 light receiving unit, 45 light shielding wall, 100 biological information detection device, 110 pulse wave information measuring unit, 113 pulse wave detection circuit, 115 pulse wave information storage unit, 120 electrocardiogram measuring unit, 123 electrocardiogram detection circuit, 125 electrocardiogram storage unit, 130 control unit, 140 analysis processing unit, 141 noise reduction unit, 142 RR interval calculation unit, 143 power calculation unit, 144 determination unit, 145 detected waveform signal storage area, 146 waveform signal storage area, 147 power waveform signal storage area, 150 output unit, 160 notification unit, 161 display unit, 163 buzzer, 170 receiving unit, 200 server system, 300 smartphone, NE network.

## Claims

1. A biological information detection device comprising:
a pulse wave information measuring unit which measures pulse wave information;
an electrocardiogram measuring unit which measures an electrocardiogram; and
a control unit which performs at least one of power supply control on the electrocardiogram measuring unit, storage control for electrocardiogram measurement result information measured by the electrocardiogram measuring unit, and notification control to output notification information about the measurement of the electrocardiogram using the electrocardiogram measuring unit, to a user, if it is determined that an arrhythmia is detected on the basis of detection result information obtained by analysis processing based on the pulse wave information.

2. The biological information detection device according to claim 1, comprising
an output unit which outputs the electrocardiogram measurement result information,
wherein the output unit
outputs the electrocardiogram measurement result information in association with the detection result information of the arrhythmia based on the pulse wave information.

3. The biological information detection device according to claim 2, wherein
the output unit
outputs body movement information of the user acquired by a body movement sensor or action information of the user determined on the basis of the body movement information, in association with the electrocardiogram measurement result information.

4. The biological information detection device according to claim 2 or 3, wherein
the output unit
outputs autonomic nerve activity information acquired on the basis of the pulse wave information, in association with the electrocardiogram measurement result information.

5. The biological information detection device according to one of claims 1 to 4, wherein
the control unit
performs at least one of the power supply control, the storage control, and the notification control, using a detection result of atrial fibrillation as the detection result information of the arrhythmia.

6. The biological information detection device according to one of claims 1 to 5, wherein
the control unit
performs at least one of the power supply control, the storage control, and the notification control, using a detection result of tachycardia as the detection result information of the arrhythmia.

7. The biological information detection device according to one of claims 1 to 6, wherein
the control unit
performs at least one of the power supply control, the storage control, and the notification control, using a detection result of bradycardia as the detection result information of the arrhythmia.

8. The biological information detection device according to one of claims 1 to 7, wherein
the control unit
performs the power supply control to start supplying electric power to the electrocardiogram measuring unit if it is determined that the arrhythmia is detected on the basis of the pulse wave information.

9. The biological information detection device according to one of claims 1 to 8, wherein
the control unit
performs the storage control to start storing the electrocardiogram measurement result information in a storage unit if it is determined that the arrhythmia is detected on the basis of the pulse wave information.

10. The biological information detection device according to one of claims 1 to 9, wherein
the control unit
performs the notification control to start outputting the notification information for prompting the measurement of the electrocardiogram using the electrocardiogram measuring unit, if it is determined that the arrhythmia is detected on the basis of the pulse wave information.

11. The biological information detection device according to one of claims 8 to 10, wherein
the control unit
halts control on the supply of the electric power, halts control on the storage of the electrocardiogram measurement result information, or halts control on the output of the notification information, on the basis of the detection result information of the arrhythmia.

12. The biological information detection device according to one of claims 1 to 11, comprising
an analysis processing unit which performs the analysis processing based on the pulse wave information and finds the detection result information of the arrhythmia.

13. The biological information detection device according to one of claims 1 to 11, comprising
a receiving unit which receives the detection result information of the arrhythmia based on the pulse wave information,
wherein the control unit
performs at least one of the power supply control, the storage control, and the notification control, on the basis of the detection result information that is received.

14. The biological information detection device according to one of claims 1 to 13, comprising
a case unit which accommodates the pulse wave information measuring unit, the electrocardiogram measuring unit, and the control unit,
wherein a first electrode for electrocardiogram measurement is provided on a surface on the side opposite to a subject, of the case unit, and
a second electrode for electrocardiogram measurement is provided on a surface on the side of the subject, of the case unit.

15. The biological information detection device according to claim 14, wherein
the case unit has a top case and a bottom case,
the first electrode is provided on a surface on the side opposite to the subject, of the top case, and
the second electrode is provided on a surface on the side of the subject, of the bottom case.

16. The biological information detection device according to one of claims 1 to 13, comprising
a light emitting unit which casts light on a subject, a light receiving unit which receives light from the subject, and a detection window for casting the light from the light emitting unit onto the subject and receiving the light from the subject,
wherein the pulse wave information measuring unit measures the pulse wave information on the basis of a signal from the light receiving unit, and
an electrode for measuring the electrocardiogram is provided in a peripheral part of the detection window.

17. The biological information detection device according to claim 16, wherein
the detection window has a protruding part for applying a pushing pressure to the subject,
the device comprises a pushing pressure restraining part which restrains the pushing pressure applied to the subject by the protruding part, and
the electrode is provided on the pushing pressure restraining part.
